# EUROPEAN PATENT SPECIFICATION

(11) **EP 3 569 163 B1**
(45) Date of publication and mention of the grant of the patent: **30.06.2021**
(21) Application number: 19170611.8
(22) Date of filing: 23.04.2019
(51) Int. Cl.: A61B 17/128

(54) **END EFFECTOR ASSEMBLIES, DRIVE SLEEVES, AND SURGICAL CLIP APPLIERS INCORPORATING THE SAME**
ENDEFFEKTORANORDNUNGEN, ANTRIEBSHÜLSEN UND CHIRURGISCHER KLAMMERAPPLIKATOR
ENSEMBLES D'EFFECTEUR D'EXTRÉMITÉ, MANCHONS D'ACTUATION ET APPLICATEURS D'AGRAFES CHIRURGICALES LES INTÉGRANT

(30) Priority: 24.04.2018 US 201862661758 P; 30.01.2019 US 201916261716
(43) Date of publication of application: 20.11.2019
(73) Proprietor: Covidien LP, Mansfield, MA 02048 (US)
(72) Inventor: DININO, Matthew A., Newington, CT 06111 (US); CRESTON, Brian J., Madison, CT 06443 (US); BARIL, Jacob C., Norwalk, CT 06851 (US); MALAVENDA, Matthew, West Haven, CT 06516 (US); BROWN, Eric, Haddam, CT 06438 (US); ZAMMATARO, Thomas, Hamden, CT 06517 (US)
(74) Representative: Birtle, Zoë Elizabeth

(56) References cited:
- EP-A1- 0 769 275
- EP-A2- 1 712 189
- EP-A2- 2 412 318
- EP-A2- 3 095 399
- WO-A1-2017/124217
- US-A- 5 725 538
- US-A- 5 797 939
- US-A- 5 833 700
- US-A1- 2006 079 913
- US-A1- 2007 073 314
- US-A1- 2017 049 446

## Description

### BACKGROUND

### Technical Field

The present disclosure relates to surgical clip appliers. More particularly, the present disclosure relates to end effector assemblies, drive sleeves, and surgical clip appliers including the same.

### Description of Related Art

Surgical clip appliers are known in the art and are used for a number of distinct and useful surgical procedures. In the case of a laparoscopic surgical procedure, access to the interior of an abdomen is achieved through narrow tubes or cannulas inserted through a small entrance incision in the skin. Minimally invasive procedures performed elsewhere in the body are often generally referred to as endoscopic procedures.

Endoscopic surgical clip appliers having various sizes (e.g., diameters), that are configured to apply a variety of diverse surgical clips, are also known in the art, and are capable of applying a single or multiple surgical clips during an entry to the body cavity. Such surgical clips are typically fabricated from a biocompatible material and are usually compressed over tissue. Once applied to tissue, the compressed surgical clip terminates the flow of fluid therethrough.

Document WO 2017/124217 A1 discloses a surgical clip applier including a handle, an elongated shaft, an end effector and a drive assembly. The distal end of the elongated shaft supports a clevis to pivotably engage the jaw members of the end effector assembly via a pivot pin.

Document US 5,725,538 A discloses a surgical clip applicator comprising a housing, a pair of handles, a slidable channel assembly and a jaw blade assembly. A clip cover which functions as tissue stop is provided.

### SUMMARY

As detailed herein and shown in the drawing figures, as is traditional when referring to relative positioning on a surgical instrument, the term "proximal" refers to the end of the apparatus or component thereof which is closer to the user and the term "distal" refers to the end of the apparatus or component thereof which is further away from the user. Further, to the extent consistent, any or all of the aspects and features detailed herein may be used in conjunction with any or all of the other aspects and features detailed herein.

The invention is defined in appended claim 1. Preferred embodiments are set forth in the dependent claims.

### BRIEF DESCRIPTION OF THE DRAWINGS

Aspects and features of the present disclosure are described in detail with reference to the drawing figures wherein like reference numerals identify similar or identical structural elements and:
FIG. 1 is a front, perspective view of a surgical clip applier provided in accordance with the present disclosure including a handle assembly having an elongated assembly engaged therewith;
FIG. 2 is front, perspective view of the surgical clip applier with the elongated assembly removed from the handle assembly;
FIG. 3A is an enlarged, side view of the handle assembly of the surgical clip applier with a portion of the housing thereof removed to illustrate the internal components and features therein, wherein the trigger is disposed in an un-actuated position;
FIG. 3B is an enlarged, side view of the handle assembly of the surgical clip applier with a portion of the housing thereof removed to illustrate the internal components and features therein, wherein the trigger is disposed in an actuated position;
FIG. 4 is a side view of the surgical clip applier with the portion of the housing of the handle assembly removed;
FIG. 5 is a side, perspective view, with portions shown transparent, of a distal portion of the elongated assembly;
FIG. 6 is a side view, with portions shown transparent, of the distal portion of the elongated assembly;
FIG. 7 is a longitudinal, cross-sectional view of the distal portion of the elongated assembly;
FIG. 8 is a perspective view of another end effector assembly configured for use with the elongated assembly;
FIG. 9 is an exploded, perspective view of the end effector assembly of FIG. 8;
FIG. 10 is a side, perspective view of a distal portion of an inner drive sleeve provided in accordance with the present disclosure shown operably coupled about the end effector assembly of FIG. 8;
FIG. 11 is a longitudinal, cross-sectional view of the distal portion of the inner drive sleeve of FIG. 10 shown operably coupled about the end effector assembly of FIG. 8;
FIG. 12 is a side view of the distal portion of the inner drive sleeve of FIG. 10 shown operably coupled about the end effector assembly of FIG. 8 and disposed in a proximal position relative thereto; and
FIG. 13 is a side view of the distal portion of the inner drive sleeve of FIG. 10 shown operably coupled about the end effector assembly of FIG. 8 and disposed in a distal position relative thereto.

### DETAILED DESCRIPTION

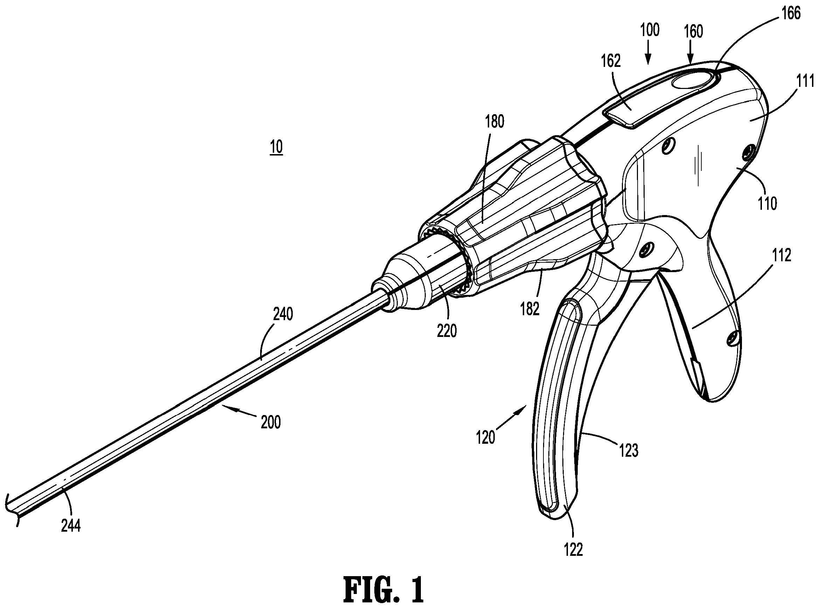

Turning to FIGS. 1-4, a surgical clip applier embodying the aspects and features of the present disclosure is shown generally identified by reference numeral 10. Surgical clip applier 10 generally includes a handle assembly 100 and an elongated assembly 200 selectively connectable to handle assembly 100. Handle assembly 100 is configured to operate elongated assembly 200 upon connection thereto, and may be configured as a sterilizable, reusable component such that handle assembly 100 may be repeatedly used with different and/or additional elongated assemblies 200 during the course of one or more surgical procedures. Elongated assembly 200 may be configured as single-use disposable component, limited-use disposable component, or reusable component, depending upon a particular purpose.

Handle assembly 100 generally includes a housing 110, an actuation mechanism 120 operably associated with housing 110, a latch assembly 160 operably associated with housing 110, and a rotating receiver assembly 180 operably coupled to a distal portion of housing 110. Housing 110 of handle assembly 100 supports and/or encloses the operating components of handle assembly 100 and defines a body portion 111 and a fixed handle portion 112 depending from body portion 111. Body portion 111 of housing 110 includes an internal pivot post 114 extending transversely within body portion 111 and a distal opening 118 through which a proximal end portion of elongated assembly 200 extends when elongated assembly 200 is engaged with handle assembly 100.

Actuation mechanism 120 is operably supported by housing 110 and includes a trigger 122, a drive bar 130, and a linkage assembly 140. Trigger 122 includes a grasping portion 123, an intermediate pivot portion 124, and a proximal extension 125. Grasping portion 123 of trigger 122 extends downwardly from body portion 111 of housing 110 in opposed relation relative to fixed handle portion 112 of housing 110. Grasping portion 123 is configured to facilitate grasping and manipulation of trigger 122. Intermediate pivot portion 124 of trigger 122 is at least partially disposed within housing 110 and defines a pivot aperture 126 that is configured to receive pivot post 114 of housing 110 so as to enable pivoting of trigger 122 about pivot post 114 and relative to housing 110, e.g., between an un-actuated position, wherein grasping portion 123 of trigger 122 is spaced-apart relative to fixed handle portion 112, and an actuated position, wherein grasping portion 123 of trigger 122 is approximated relative to fixed handle portion 112.

Proximal extension 125 of trigger 122 is disposed on an opposite side of intermediate pivot portion 124 and, thus, pivot post 114, as compared to grasping portion 123 of trigger 122. As such, pivoting of grasping portion 123 to rotate in one direction, e.g., proximally towards fixed handle portion 112, pivots proximal extension 125 to rotate in the opposite direction, e.g., distally.

Linkage assembly 140 includes a first linkage 142, a second linkage 144, and a third linkage 146. First linkage 142 is pivotably coupled to proximal extension 125 of trigger 122 towards a first end 143a of first linkage 142. Second and third linkages 144, 146, respectively, are each pivotably coupled to a second end 143b of first linkage 142 at respective first ends 145a, 147a of second and third linkages 144, 146. A second end 145b of second linkage 144 is pivotably coupled to drive bar 130, while a second end 147b of third linkage 146 is pivotably coupled to body portion 111 of housing 110. Thus, the pivot point between first linkage 142 and proximal extension 125 of trigger 122, the pivot point between first linkage 142 and second and third linkages 144, 146, respectively, and the pivot point between second linkage 144 and drive bar 130 are movable pivot points (e.g., movable relative to housing 110), while the pivot point between third linkage 146 and housing 110 is a fixed pivot point (e.g., fixed relative to housing 110).

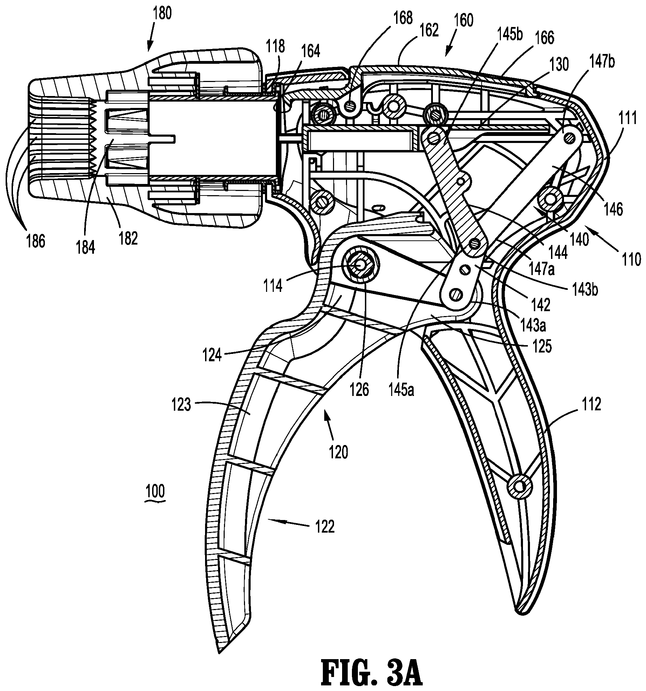

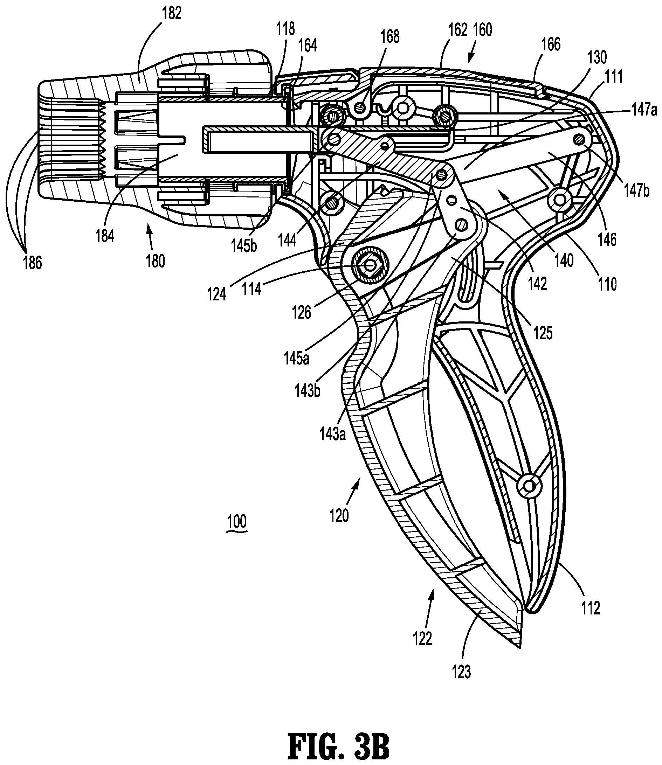

Upon actuation of trigger 122, e.g., proximal pivoting of grasping portion 123 of trigger 122, proximal extension 125 is moved in a counter-clockwise direction (from the orientation illustrated in FIG. 3), thereby urging first linkage 142 towards drive bar 130. This movement of first linkage 142 towards drive bar 130, in turn, urges first ends 145a, 147a of second and third linkages 144, 146, respectively, towards drive bar 130 to, in turn, urge second end 145b of second linkage 144 distally such that drive bar 130 is translated distally through body portion 111 of housing 110. A biasing spring (not shown) may be provided to bias trigger 122 towards an un-actuated positon, thereby biasing drive bar 130 proximally.

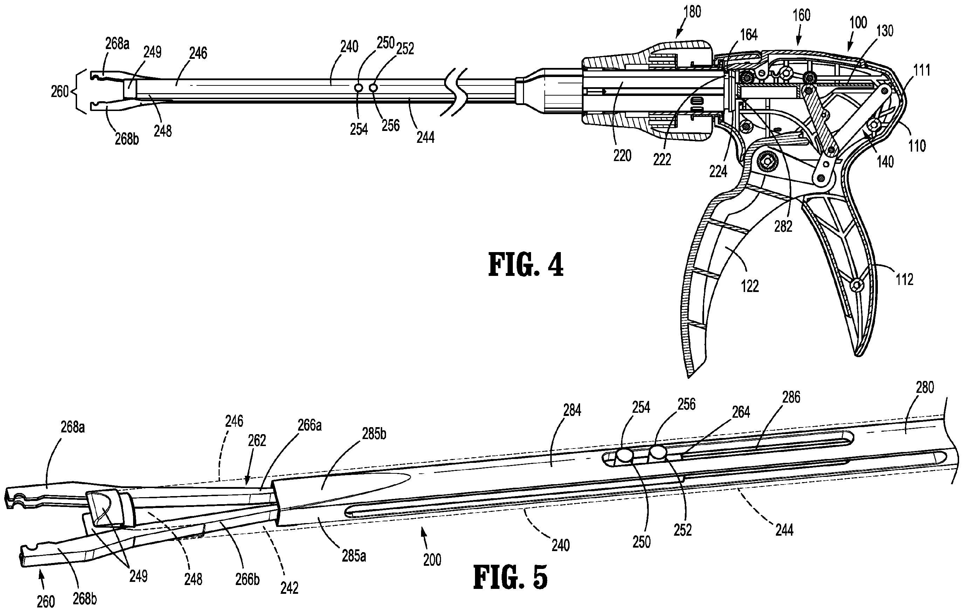

Drive bar 130 is slidably disposed within body portion 111 of housing 110 in longitudinal alignment with proximal portion 282 of inner drive sleeve 280 of elongated assembly 200 (see FIG. 4) when elongated assembly 200 is engaged with handle assembly 100 such that distal sliding of drive bar 130 through body portion 111 of housing urges drive bar 130 into contact with proximal portion 282 of inner drive sleeve 280 to thereby translate inner drive sleeve 280 distally, e.g., to apply, form or close a surgical clip supported at end effector assembly 260 of elongated assembly 200, as detailed below.

Latch assembly 160 is configured to facilitate releasable locking engagement of elongated assembly 200 with handle assembly 100. Latch assembly 160, more specifically, includes a pivoting lever arm 162 operably disposed on and extending into body portion 111 of housing 110. Lever arm 162 includes an engagement finger 164 disposed towards one end thereof and a manipulatable portion 166 disposed towards the other end thereof with a pivot portion 168 disposed therebetween. Thus, upon depression of manipulatable portion 166 into housing 110 from a locked position to an unlocked position, engagement finger 164 is withdrawn upwardly and, upon release of manipulatable portion 166 and return thereof to the locked position, engagement finger 164 is returned downwardly. A torsion spring (not shown) disposed about pivot portion 168, or other suitable biasing spring in any suitable position, may be provided to bias lever arm 162 towards the locked position, although other configurations are also contemplated.

Rotating receiver assembly 180 is configured to receive a proximal end portion of elongated assembly 200 and to enable selective rotation thereof relative to housing 110. Rotating receiver assembly 180 includes a rotation knob 182 rotatably coupled to body portion 111 of housing 110 and extending distally therefrom. Rotation knob 182 defines a lumen 184 extending therethrough in communication with distal opening 118 of body portion 111 of housing 110 to enable insertion of a proximal portion of elongated assembly 200 therethrough and into operable engagement within housing 110. Rotation knob 184 defines channels 186 disposed on an interior surface thereof and arranged annularly about lumen 184 to enable rotatable coupling of elongated assembly 200 therewith, as detailed below.

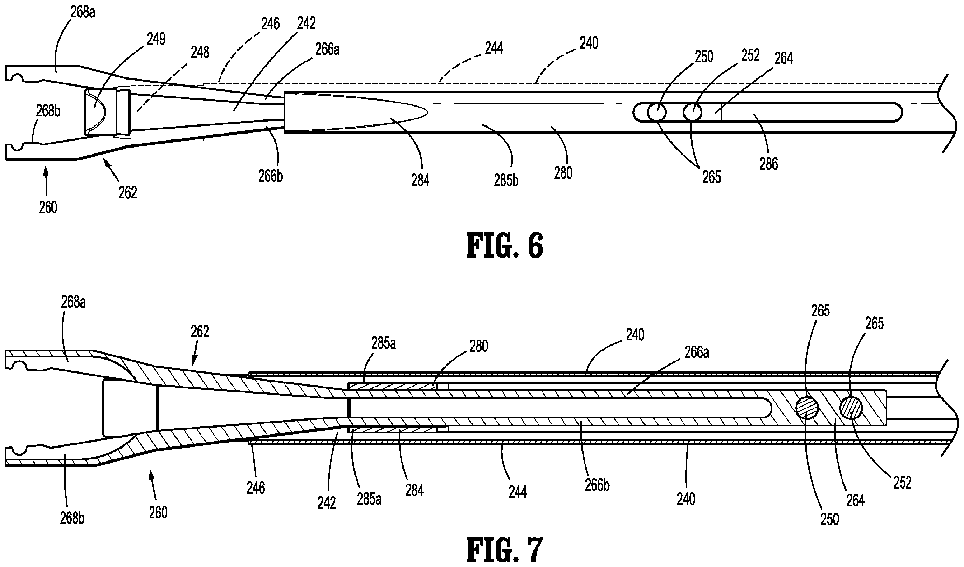

With additional reference to FIGS. 5-7, elongated assembly 200 generally includes a proximal hub 220, an elongated shaft 240 extending distally from proximal hub 220, an end effector assembly 260 disposed towards a distal end portion of elongated shaft 240, and an inner drive sleeve 280 slidably disposed through proximal hub 220 and elongated shaft 240 and configured for operable coupling between handle assembly 100 and end effector assembly 260 when elongated assembly 200 is engaged with handle assembly 100 to enable firing of a surgical clip (not shown) about tissue.

Proximal hub 220 is configured for insertion through lumen 184 of rotation knob 182 and into body portion 111 of housing 110. Proximal hub 220 defines an annular recess 222 towards the proximal end thereof and a chamfered proximal edge 224. Thus, upon insertion of proximal hub 220 through lumen 184 of rotation knob 182 and into body portion 111 of housing 110, chamfered proximal edge 224 cams engagement finger 164 of latch assembly 160 over the outer surface of proximal hub 220 until engagement finger 164 is disposed in alignment with annular recess 222, wherein engagement finger 164 falls into engagement within annular recess 222 to engage proximal hub 220 and, thus, elongated assembly 200, with handle assembly 100. As can be appreciated, in order to disengage and remove elongated assembly 200 from handle assembly 100, manipulatable portion 166 of latch assembly 160 is depressed into housing 110 to withdraw engagement finger 164 from annular recess 222 and enable elongated assembly 200 to be pulled distally and removed from handle assembly 100. Proximal hub 220 may further include a lock tab 226 extending along a portion of the length thereof and configured for receipt within one of the channels 186 defined within rotation knob 182 to rotationally fix elongated assembly 20 relative to rotation knob 182 upon insertion therein.

Elongated shaft 240 extends distally from proximal hub 220 and defines a longitudinal lumen 242 extending therethrough. Elongated shaft 240 further includes a body 244 and a bifurcated distal portion 246 including a pair of radially-opposed flanges 248 extending distally from body 244. Opposed flanges 248 define tissue stops 249 configured to inhibit passage of tissue into the space defined therebetween, as detailed below.

End effector assembly 260 of elongated assembly 200 is formed as a monolithic component of a single piece of material, e.g., via stamping or other suitable manufacturing process, and includes a jaws component 262 having a proximal base 264, a pair of spaced-apart arms 266a, 266b extending distally from proximal base 264, and a jaw 268a, 268b disposed at the free distal end of each arm 266a, 266b, respectively.

Proximal base 264 of jaws component 262 defines pair of apertures 265 extending transversely therethrough and in longitudinal alignment with one another, although greater or fewer apertures or otherwise arranged apertures are also contemplated. Apertures 265 are configured for receipt of pins 250, 252 which extend transversely through elongated shaft 240 and at least partially into opposed pairs of apertures 254, 256, respectively, defined transversely through elongated shaft 240. The portions of pins 250, 252 extending into or through apertures 254, 256 may be welded to elongated shaft 240 or otherwise engaged thereto to fix pins 250, 252 and, thus, proximal base 264 of jaws component 262 relative to elongated shaft 240.

Spaced-apart arms 266a, 266b of jaws component 262 extend distally from proximal base 264 to jaws 268a, 268b, respectively, and are resiliently flexible from an at-rest position, wherein spaced-apart arms 266a, 266b are angled apart from one another to define an increasing distance therebetween in the proximal-to-distal direction, to a flexed position, wherein spaced-apart arms 266a, 266b are closer to one another and disposed in a more-parallel orientation or angled towards one another. Spaced-apart arms 266a, 266b are oriented 90 degrees offset from flanges 248 of elongated shaft 240 to enable the portions of spaced-apart arms 266a, 266b disposed between flanges 248 to extend radially outwardly beyond the radial dimension of elongated shaft 240 in the at-rest position thereof without interference from flanges 248. This configuration also positions tissue stops 249 on the lateral sides of spaced-apart arms 266a, 266 to inhibit tissue ingress into the space defined between spaced-apart arms 266a, 266b.

Jaws 268a, 268b, as noted above, are disposed at the free distal ends of spaced-apart arms 266a, 266b, respectively. Jaws 268a, 268b may define transverse notches 270, longitudinal slots 272, and/or other suitable features to facilitate retention of legs of a surgical clip (not shown) therein. Jaws 268a, 268b are moved from a spaced-apart position to an approximated position upon movement of spaced-apart arms 266a, 266b from the at-rest position to the flexed position to thereby form a surgical clip held between jaws 268a, 268b about tissue disposed between jaws 268a, 268b. End effector assembly 260, in embodiments, may be configured to form surgical clips similar to those shown and described in U.S. Patent No. 4,834,096.

Inner drive sleeve 280 defines a proximal portion 282 (FIG. 4) and a distal portion 284. Proximal portion 282 of inner drive sleeve 280 is configured for positioning adjacent a distal end of drive bar 130 of handle assembly 100 when elongated assembly 200 is engaged with handle assembly 100 (see FIG. 4) such that distal translation of drive bar 130 through housing 110 (e.g., upon actuation of trigger 122), urges drive bar 130 into contact with inner drive sleeve 280 to translate inner drive sleeve 280 distally through elongated shaft 240 of elongated assembly 200.

Distal portion 284 of inner drive sleeve 280 is slidably disposed about at least a proximal portion of jaws component 262 of end effector assembly 260 and defines a rectangular transverse cross-sectional configuration having a pair of narrow sides 285a and a pair of wide sides 285b. Opposed longitudinally-extending slots 286 are defined through wide sides 285b of distal portion 284 of inner drive sleeve 280 in alignment with one another. Slots 286 enable passage of pins 250, 252 therethrough while still enabling sliding of distal portion 284 of inner drive sleeve 280 through elongated shaft 240 and about end effector assembly 260. Distal portion 284 of inner drive sleeve 280 is oriented such that spaced-apart arms 266a, 266b of jaws component 262 are disposed adjacent opposed narrow sides 285a of distal portion 284 with, in embodiments, the width of opposed narrow sides 285a generally approximating the width of spaced-apart arms 266a, 266b to inhibit relative lateral motion between spaced-apart arms 266a, 266b, thereby inhibiting splay between jaws 268a, 268b.

Wide sides 285b of distal portion 284 of inner drive sleeve 280 define heights greater than the minimum distance between spaced-apart arms 266a, 266b but less than the maximum distance between spaced-apart arms 266a, 266b such that distal sliding of distal portion 284 of inner drive sleeve 280 about jaws component 262, e.g., in response to actuation of trigger 122, cams narrow sides 285a about the exterior surfaces of spaced-apart arms 266a, 266b to urge spaced-apart arms 266a, 266b towards one another from the at-rest position towards the flexed position, thereby moving jaws 268a, 268b from the spaced-apart position towards the approximated position to form or close a surgical clip positioned therebetween about tissue disposed between jaws 268a, 268b. Upon release or return of trigger 122, inner drive sleeve 280 is returned proximally, allowing spaced-apart arms 266a, 266b to resiliently return towards the at-rest position, thereby returning jaws 268a, 268b towards the spaced-apart position to enable loading of a subsequent surgical clip for formation or closing about tissue. A biasing spring (not shown) associated with elongated assembly 200 may be provided to bias inner drive sleeve 280 proximally such that, upon release of trigger 122, inner drive sleeve 280 is returned proximally. Other suitable biasing configurations are also contemplated.

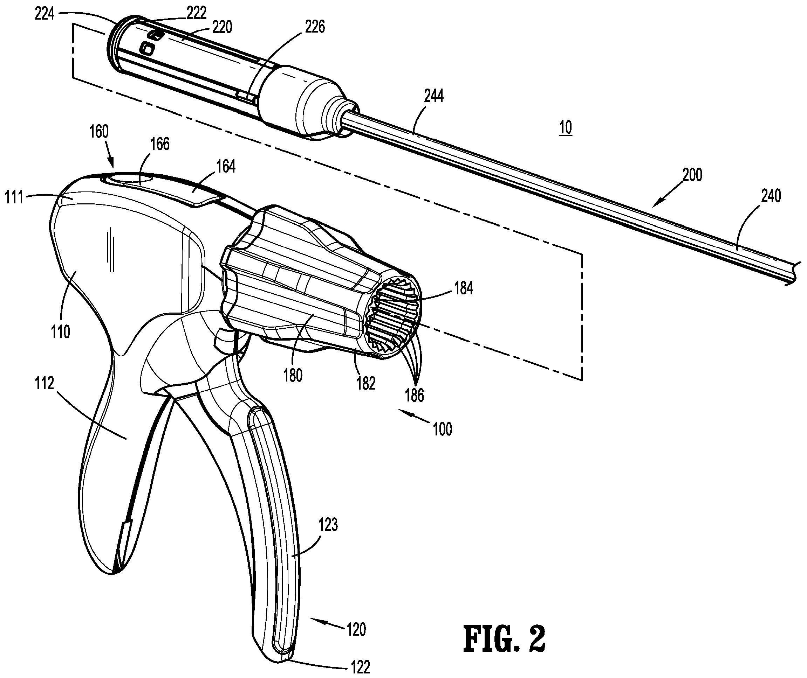

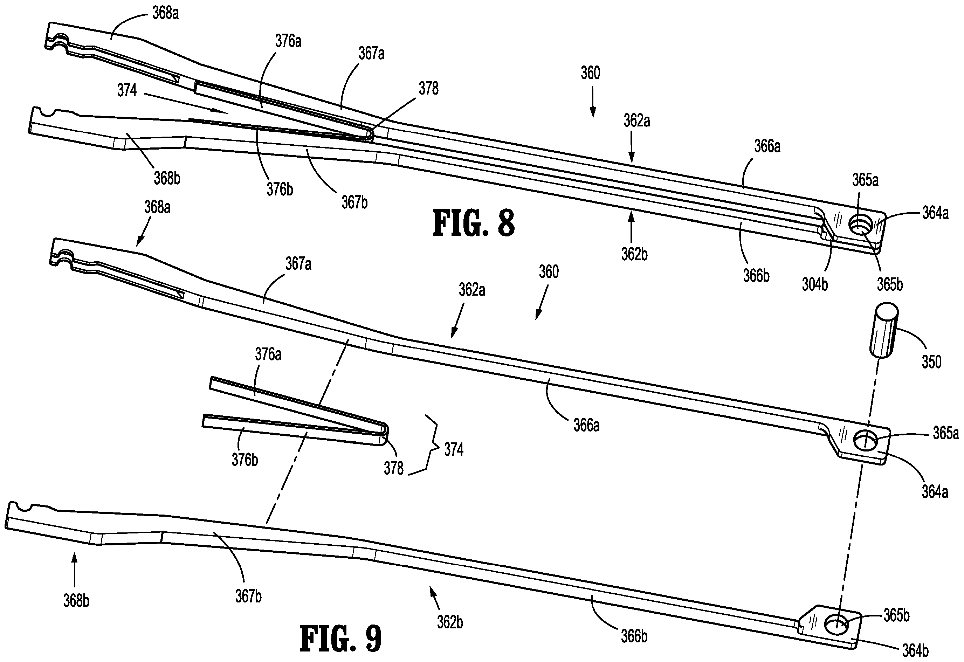

Turning to FIGS. 8 and 9, another embodiment of an end effector assembly provided in accordance with the present disclosure and configured for use with elongated assembly 200 (FIGS. 2 and 4-7) is shown generally identified by reference numeral 360. End effector assembly 360 includes first and second jaw components 362a, 362b, each including a proximal base 364a, 364b, an arm 366a, 366b extending distally from the respective proximal base 364a, 364b, and a jaw 368a, 368b disposed at the free distal end of the respective arm 366a, 366b. End effector assembly 360 further includes a leaf spring 374 including first and second legs 376a, 376b interconnected by a hinge 378. End effector assembly 360 may be similar to or include any of the features of end effector assembly 260 (FIGS. 5-7), except where specifically contradicted below.

Rather than providing a single, monolithic component as with jaws component 262 of end effector assembly 260 (see FIGS. 5-7), end effector assembly 360 includes separate first and second jaw components 362a, 362b. Proximal bases 364a, 364b of jaw components 362a 362b, respectively, are offset relative to respective arms 366a, 366b thereof such that jaw proximal bases 364a, 364b of jaw components 362a, 362b may be positioned in side-by-side relation relative to one another with arms 366a, 366b disposed in opposing alignment with one another. Proximal bases 364a, 364b further define aligned apertures 365a, 365b, respectively, extending transversely therethrough that are configured for receipt of a pin 350 to longitudinally fix and pivotably couple proximal bases 364a, 364b within the elongated shaft 240 (FIGS. 5-7), similarly as detailed above with respect to pins 250, 252, proximal base 264 of jaws component 262, and elongated shaft 240 (see FIGS. 5-7). Pin 350 also serves to pivotably couple proximal bases 364a, 364b with one another.

Arms 366a, 366b of end effector assembly 360 extend distally from respective proximal bases 364a, 364b. Arms 366a, 366b are identical to one another, with one arm 366a, 366b being inverted to face the other arm 366a, 366b. Arms 366a, 366b are substantially rigid in that arms 366a, 366b are not required to flex during proper operation of end effector assembly 360. Rather, arms 366a, 366b are pivotable relative to one another about pin 350 from a further-spaced position to a closer-together position. Each arm 366a, 366b includes a distal segment 367a, 367b, wherein jaws 368a, 368b extend distally from distal segments 367a, 367b of arms 366a, 366b, respectively.

Jaws 368a, 368b of end effector assembly 360 are similar to and may include any of the features of jaws 268a, 268b of end effector assembly 260, detailed above (see FIGS. 5-7), and are configured to move from a spaced-apart position towards an approximated position in response to movement of arms 366a, 366b from the further-spaced position towards the closer-together position to form or close a surgical clip about tissue.

Leaf spring 374 is configured for positioning between distal segments 367a, 367b of arms 366a, 366b with first and second legs 376a, 376b of leaf spring 374 abutting inwardly-facing surfaces of distal segments 367a, 367b of arms 366a, 366b, respectively, and extending distally from hinge 378. As such, leaf spring 374 biases arms 366a, 366b towards the further-spaced position and, thus, jaws 368a, 368b towards the spaced-apart position. First and second legs 376a, 376b of leaf spring 374 may be adhered or otherwise secured in engagement with the inwardly-facing surfaces of distal segments 367a, 367b of arms 366a, 366b, or may be retained therein via inner drive sleeve 280 (FIGS. 5-7) being disposed at least partially about distal segments 367a, 367b.

With additional reference to FIGS. 5-7, in use, distal sliding of distal portion 284 of inner drive sleeve 280 about jaw components 362a, 362b, e.g., in response to actuation of trigger 122 (FIG. 1), cams narrow sides 285a about the exterior surfaces of arms 366a, 366b to urge arms 366a, 366b to pivot about pin 350 towards one another from the further-spaced position towards the closer-together position, thereby moving jaws 368a, 368b from the spaced-apart position towards the approximated position to form or close a surgical clip positioned therebetween about tissue disposed between jaws 368a, 368b. The pivoting of arms 366a, 366b about pin 350 towards the closer-together position urges legs 376a, 376b of leaf spring 374 towards one another, against the bias of leaf spring 374. As such, upon release or return of trigger 122 (FIG. 1), inner drive sleeve 280 is returned proximally and jaws 368a, 368b and arms 366a, 366b are returned apart from one another towards the spaced-apart and further-spaced positions, respectively, under the bias of leaf spring 374 to enable loading of a subsequent surgical clip for formation or closure about tissue.

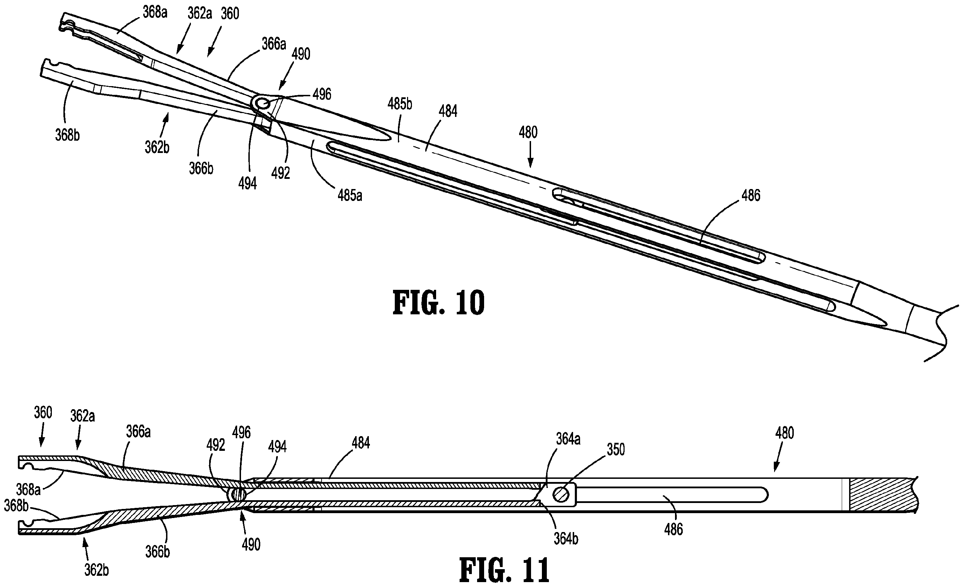

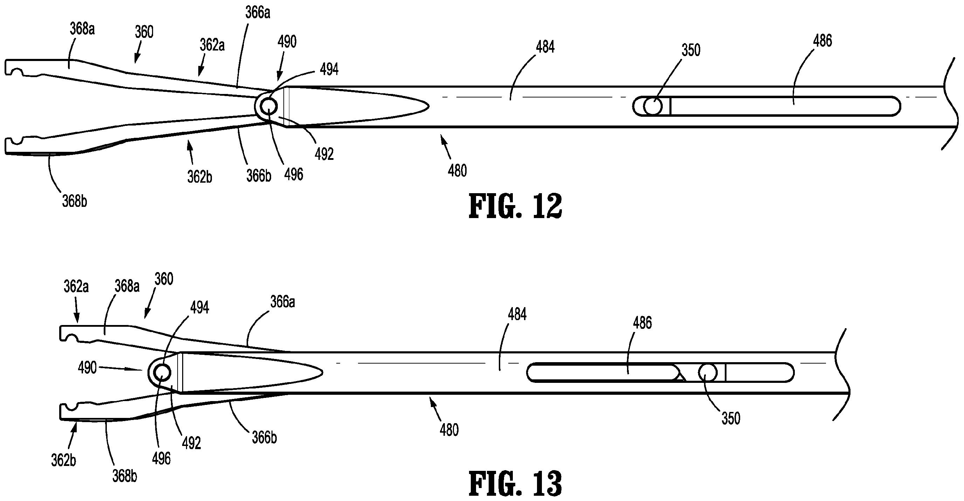

Turning to FIGS. 10-13, another embodiment of an inner drive sleeve provided in accordance with the present disclosure and configured for use with end effector assembly 260 of elongated assembly 200 (FIGS. 2 and 4-7) or end effector assembly 360 (as shown; see also FIGS. 8 and 9) is shown generally identified by reference numeral 480. Inner drive sleeve 480 includes a proximal portion (not shown) similar to proximal portion 282 of inner drive sleeve 280 (FIG. 4), and a distal portion 484.

Distal portion 484 of inner drive sleeve 480 is similar to distal portion 284 of inner drive sleeve 280 (FIGS. 5-7), slidably disposed about jaw components 362a, 362b of end effector assembly 360, and defines a rectangular transverse cross-sectional configuration. Opposed longitudinally-extending slots 486 are defined through wide sides 485b of distal portion 484 of inner drive sleeve 480 in alignment with one another. Slots 486 enable passage of pin 350 therethrough while still enabling sliding of distal portion 484 of inner drive sleeve 480 through elongated shaft 240 (FIGS. 5-7) and about end effector assembly 360.

Distal portion 484 of inner drive sleeve 480 further includes a clevis 490 extending distally from the distal ends of wide sides 485b of distal portion 484. Clevis 490, more specifically, includes a pair of clevis flanges 492 extending from wide sides 485b of distal portion 484 of inner drive sleeve 480 in spaced-apart relation relative to one another. Each clevis flange 492 defines an aperture 494 therethrough that is disposed in alignment with the aperture 494 of the other clevis flange 492. A pin 496 is received within apertures 494 and extends transversely between clevis flanges 492.

When end effector assembly 360 (or other suitable end effector assembly, e.g., end effector assembly 260 (FIGS. 5-7)) is assembled with inner drive sleeve 480, the pin 350 coupling proximal bases 364a, 364b of arms 366a, 366b extends through slots 486 of inner drive sleeve 480 to enable engagement of pin 350 with elongated shaft 240 (FIGS. 5-7) to pivotably couple arms 366a, 366b with one another and engage proximal bases 364a, 364b of arms 362a, 362b with elongated shaft 240 (FIGS. 5-7). Arms 366a, 366b extend distally from proximal bases 364a, 364b through inner drive sleeve 480, ultimately exiting inner drive sleeve 480 with arms 366a, 366b disposed on opposing sides of pin 496. Jaws 368a, 368b extend distally from arms 366a, 366b on either side of pin 496.

Pin 496 of clevis 490 of distal portion 484 of inner drive sleeve 480 defines a suitable diameter and is positioned, in the proximal position of inner drive sleeve 480, between arms 366a, 366b so as to function as a wedge maintaining arms 366a, 366b in the further-spaced position and, thus, jaws 368a, 368b in the spaced-apart position. As can be appreciated, as pin 496 is moved distally relative to the pivot point of jaws 368a, 368b, e.g., the location of pin 350, jaws 368a, 386b are permitted to pivot further towards one another whereas proximal movement of pin 496 relative to the pivot point of jaws 368a, 368b urges jaws 368a, 368b to pivot further apart from one another.

In use, distal sliding of distal portion 484 of inner drive sleeve 480 about arms 366a, 366b, e.g., in response to actuation of trigger 122 (FIG. 1), cams narrow sides 485a of inner drive sleeve 480 about the exterior surfaces of arms 366a, 366b to urge arms 366a, 366b to pivot about pin 350 towards one another from the further-spaced position towards the closer-together position, thereby moving jaws 368a, 368b from the spaced-apart position towards the approximated position to form or close a surgical clip positioned therebetween about tissue disposed between jaws 368a, 368b. This distal sliding of inner drive sleeve 480 relative to end effector assembly 360 moves pin 496 distally such that, as noted above, jaws 368a, 368b are permitted to pivot to the approximated position.

Upon release or return of trigger 122 (FIG 1), inner drive sleeve 480 is returned proximally and, thus, pin 496 is likewise returned proximally. As pin 496 is moved proximally towards the pivot point between jaws 368a, 368b, pin 496 eventually contacts the inwardly-facing surfaces of arms 366a, 366b, thereby functioning as a wedge to urge arms 366a, 366b to pivot apart from one another, thus urging jaws 368a, 368b to pivot towards the spaced-apart positon. Thus, pin 496 serves to return jaws 368a, 368b to the spaced-apart position upon release or return of trigger 122 (FIG. 1) to enable loading of a subsequent surgical clip for formation or closure about tissue. More specifically, pin 496 returns jaws 368a, 368b to the spaced-apart position without imparting a return biasing force that is required to be overcome in order to approximate jaws 368a, 368b. Thus, utilizing pin 496 to return jaws 368a, 368b to the spaced-apart position provides a decreased overall actuation force for approximating jaws 368a, 368b.

## Claims

1. A surgical clip applier (10), comprising:
a handle assembly (100) including a housing (110) and a trigger operably (122) coupled to the housing;
an elongated assembly (200) extending distally from the handle assembly, the elongated assembly including:
an outer shaft (240) including a body and defining a longitudinal lumen (242) extending therethrough;
an end effector assembly (260, 360) disposed partially within and extending distally from the outer shaft (240), the end effector assembly including a jaws component (262, 362) engaged to the outer shaft at a proximal portion (264) thereof and including first and second spaced-apart arms (266a, 266b, 366a, 366b) extending distally from the proximal portion, the jaws component further including first and second jaws (268a, 268b, 368a, 368b) disposed at free ends of the first and second spaced-apart arms, respectively; and
an inner drive sleeve (280, 480) slidably disposed about the end effector assembly (260, 360) within the outer shaft (240), wherein the inner drive sleeve is slidably disposed about the first and second spaced-apart arms (266a, 266b, 366a, 366b) and is movable from a proximal position to a distal position to cam the first and second spaced-apart arms towards one another, thereby moving the first and second jaws (268a, 268b, 368a, 368b) from a spaced-apart position to an approximated position to apply a surgical clip about tissue disposed between the first and second jaws,
**characterized in that** the outer shaft (240) comprises a pair of flanges (248) extending distally from opposed sides of the body, the opposed flanges defining tissue stops (249) at free ends thereof, wherein the first and second spaced-apart arms (266a, 266b, 366a, 366b) extend distally between the opposed flanges of the outer shaft.

2. The surgical clip applier according to claim 1, wherein the jaws component (262) is monolithically formed from a single piece of material.

3. The surgical clip applier according to claim 1 or claim 2, wherein the jaws component (262) is stamped.

4. The surgical clip applier according to claim 2, wherein the proximal portion (264) of the jaws component is pinned to the outer shaft (240) via at least one pin (250, 252).

5. The surgical clip applier according to claim 4, wherein the at least one pin (250, 252) extends through opposed slots (286) defined within the inner drive sleeve (280).

6. The surgical clip applier according to any preceding claim, wherein the first and second arms (266a, 266b) are resiliently flexible from an at-rest position to a flexed position in response to movement of the inner drive sleeve (280) from the proximal position to the distal position to thereby move the first and second jaws (268a, 268b) from the spaced-apart position to the approximated position.

7. The surgical clip applier according to claim 1, wherein the jaws component (362) comprises first and second jaw components (362a, 362b) pivotably coupled to one another and the outer shaft (240) at proximal portions (364a, 364b) of the first and second jaw components, the first and second jaw components including, respectively, the first and second arms (366a, 366b) extending distally from the respective proximal portions thereof, and the first and second jaws (368a, 368b) being disposed at free ends of the first and second arms, respectively.

8. The surgical clip applier according to claim 7, wherein the first and second jaw components (362a, 362b) are identical to one another.

9. The surgical clip applier according to claim 7 or 8, wherein the first and second jaw components (362a, 362b) are substantially rigid.

10. The surgical clip applier according to claim 7, 8 or 9, further comprising a leaf spring (374) disposed between the first and second arms (366a, 366b) configured to bias the first and second jaws (368a, 368b) towards the spaced-apart position.

11. The surgical clip applier according to claim 10, wherein the leaf spring (374) includes a hinge (378) and first and second legs (376a, 376b) extending distally from the hinge, the first leg engaged with the first arm (366a) and the second leg engaged with the second arm (366b).

12. The surgical clip applier according to any of claims 7 to 11, wherein the proximal portions (364a, 364b) of the first and second jaw components (362a, 362b) are pivotably coupled to one another and the outer shaft (240) via a pin (350), and wherein the pin (350) extends through opposed slots (286, 486) defined within the inner drive sleeve (280, 480).

13. The surgical clip applier of any preceding claim, wherein the inner drive sleeve (480) includes a distal body portion (484) and a clevis (490) extending distally from the distal body portion, the clevis including first and second spaced-apart flanges (492) and a pin (496) extending transversely between the first and second flanges, whereby, upon return of the inner drive sleeve (480) from the distal position to the proximal position, the pin (496) is wedged between the first and second arms (366a, 366b) to urge the first and second jaws (368a, 368b) from the approximated position back to the spaced-apart position.

14. The surgical clip applier according to claim 13, wherein the inner drive sleeve (480) defines a rectangular transverse, cross-sectional configuration having opposed narrow sides (485a) and opposed wide sides (485b).

15. The surgical clip applier according to claim 14, wherein the flanges (492) of the clevis (490) extend from the opposed wide sides (485b) of the inner drive sleeve (480).

## Patentansprüche

1. Anbringvorrichtung für chirurgische Klammern (10), die Folgendes umfasst:
eine Griffanordnung (100), die ein Gehäuse (110) und einen Auslöser (122) einschließt, der mit dem Gehäuse wirkgekoppelt ist;
eine längliche Anordnung (200), die sich distal aus der Griffanordnung erstreckt, wobei die längliche Anordnung Folgendes einschließt:
einen äußeren Schaft (240), der einen Körper einschließt und ein sich dahindurch erstreckendes Längslumen (242) definiert;
eine Greiforgananordnung (260, 360), die teilweise innerhalb des äußeren Schafts (240) eingerichtet ist und sich aus diesem distal erstreckt, wobei die Greiforgananordnung eine Backenkomponente (262, 362) einschließt, die mit dem äußeren Schaft an einem proximalen Abschnitt (264) davon in Eingriff steht und erste und zweite beabstandete Arme (266a, 266b, 366a, 366b) einschließt, die sich aus dem proximalen Abschnitt distal erstrecken, wobei die Backenkompenente ferner erste und zweite Backen (268a, 268b, 368a, 368b) einschließt, die an freien Enden der ersten beziehungsweise der zweiten beabstandeten Arme eingerichtet sind; und
eine innere Antriebsmuffe (280, 480), die um die Greiforgananordnung (260, 360) innerhalb des äußeren Schafts (240) schiebbar eingerichtet ist, wobei die innere Antriebsmuffe um die ersten und die zweiten beabstandeten Arme (266a, 266b, 366a, 366b) schiebbar eingerichtet ist und von einer proximalen Position in eine distale Position bewegbar ist, um die ersten und die zweiten beabstandeten Arme zueinander zu führen, wobei sie dadurch die ersten und die zweiten Backen (268a, 268b, 368a, 368b) von einer beabstandeten Position in eine angenäherte Position bewegt, um eine chirurgische Klammer um Gewebe anzubringen, das zwischen der ersten und der zweiten Backe eingerichtet ist, **dadurch gekennzeichnet, dass** der äußere Schaft (240) ein Paar Flansche (248) umfasst, die sich aus gegenüberliegenden Seiten des Körpers distal erstrecken, wobei die gegenüberliegenden Flansche Gewebeanschläge (249) an freien Enden davon definieren, wobei sich die ersten und die zweiten beabstandeten Arme (266a, 266b, 366a, 366b) zwischen den gegenüberliegenden Flanschen des äußeren Schafts distal erstrecken.

2. Anbringvorrichtung für chirurgische Klammern nach Anspruch 1, wobei die Backenkomponente (262) aus einem einzigen Materialstück monolithisch ausgebildet ist.

3. Anbringvorrichtung für chirurgische Klammern nach Anspruch 1 oder 2, wobei die Backenkomponente (262) gestanzt ist.

4. Anbringvorrichtung für chirurgische Klammern nach Anspruch 2, wobei der proximale Abschnitt (264) der Backenkomponente über wenigstens einen Stift (250, 252) an dem äußeren Schaft (240) befestigt ist.

5. Anbringvorrichtung für chirurgische Klammern nach Anspruch 4, wobei der wenigstens eine Stift (250, 252) sich durch gegenüberliegende Schlitze (286) erstreckt, die innerhalb der inneren Antriebsmuffe (280) definiert sind.

6. Anbringvorrichtung für chirurgische Klammern nach einem vorhergehenden Anspruch, wobei der erste und der zweite beabstandete Arm (266a, 266b) als Reaktion auf eine Bewegung der inneren Antriebsmuffe (280) von der proximalen Position in die distale Position von einer Ruheposition in eine geknickte Position federnd flexibel sind, um dadurch die erste und die zweite Backe (268a, 268b) von der beabstandeten Position in die angenäherte Position zu bewegen.

7. Anbringvorrichtung für chirurgische Klammern nach Anspruch 1, wobei die Backenkomponente (362) eine erste und eine zweite Backenkomponente (362a, 362b) umfasst, die miteinander und dem äußeren Schaft (240) an proximalen Abschnitten (364a, 364b) der ersten und der zweiten Backenkomponente schwenkbar gekoppelt sind, wobei die erste und die zweite Backenkomponente den ersten beziehungsweise den zweiten Arm (366a, 366b) einschließen, die sich distal aus den jeweiligen proximalen Abschnitten davon erstrecken, und wobei die erste und die zweite Backe (368a, 368b) an freien Enden des ersten beziehungsweise des zweiten Arms eingerichtet sind.

8. Anbringvorrichtung für chirurgische Klammern nach Anspruch 7, wobei die erste und die zweite Backenkomponente (362a, 362b) identisch zueinander sind.

9. Anbringvorrichtung für chirurgische Klammern nach Anspruch 7 oder 8, wobei die erste und die zweite Backenkomponente (362a, 362b) im Wesentlichen unbiegsam sind.

10. Anbringvorrichtung für chirurgische Klammern nach Anspruch 7, 8 oder 9, die ferner eine Blattfeder (374) umfasst, die zwischen dem ersten und dem zweiten Arm (366a, 366b) eingerichtet ist, die konfiguriert sind, um die erste und die zweite Backe (368a, 368b) zu der beabstandeten Position vorzuspannen.

11. Anbringvorrichtung für chirurgische Klammern nach Anspruch 10, wobei die Blattfeder (374) ein Scharnier (378) und erste und zweite Schenkel (376a, 376b) einschließt, die sich aus dem Scharnier distal erstrecken, wobei der erste Schenkel mit dem ersten Arm (366a) in Eingriff steht und der zweite Schenkel mit dem zweiten Arm (366b) in Eingriff steht.

12. Anbringvorrichtung für chirurgische Klammern nach einem der Ansprüche 7 bis 11, wobei die proximalen Abschnitte (364a, 364b) der ersten und der zweiten Backenkomponente (362a, 362b) über einen Stift (350) miteinander und dem äußeren Schaft (240) schwenkbar gekoppelt sind, und wobei der Stift (350) sich durch gegenüberliegende Schlitze (286, 486) erstreckt, die innerhalb der inneren Antriebsmuffe (280, 480) definiert sind.

13. Anbringvorrichtung für chirurgische Klammern nach einem vorhergehenden Anspruch, wobei die innere Antriebsmuffe (480) einen distalen Körperabschnitt (484) und einen Gabelkopf (490) einschließt, der sich aus dem distalen Körperabschnitt distal erstreckt, wobei der Gabelkopf einen ersten und einen zweiten beabstandeten Flansch (492) und einen Stift (496) einschließt, der sich zwischen dem ersten und dem zweiten Flansch quer erstreckt, wodurch der Stift (496) bei Rückkehr der inneren Antriebsmuffe (480) von der distalen Position in die proximale Position zwischen dem ersten und dem zweiten Arm (366a, 366b) verkeilt ist, um die erste und die zweite Backe (368a, 368b) von der angenäherten Position zurück in die beabstandete Position zu drängen.

14. Anbringvorrichtung für chirurgische Klammern nach Anspruch 13, wobei die innere Antriebsmuffe (480) eine rechteckige quere Querschnittskonfiguration definiert, die gegenüberliegende Schmalseiten (485a) und gegenüberliegende Breitseiten (485b) aufweist.

15. Anbringvorrichtung für chirurgische Klammern nach Anspruch 14, wobei sich die Flansche (492) des Gabelkopfs (490) aus den gegenüberliegenden Breitseiten (485b) der inneren Antriebsmuffe (480) erstrecken.

## Revendications

1. Applicateur d'agrafes chirurgicales (10), comprenant :
un ensemble poignée (100) comportant un boîtier (110) et une détente (122) accouplée de manière fonctionnelle au boîtier ;
un ensemble allongé (200) s'étendant de manière distale à partir de l'ensemble poignée, l'ensemble allongé comportant :
une gaine externe (240) comportant un corps et définissant une lumière longitudinale (242) s'étendant à travers celle-ci ;
un ensemble effecteur terminal (260, 360) disposé partiellement à l'intérieur de la gaine externe et s'étendant de manière distale à partir de la gaine externe (240), l'ensemble effecteur terminal comportant un composant de mâchoires (262, 362) en prise avec la gaine externe au niveau d'une partie proximale (264) de celui-ci et comportant de premier et second bras (266a, 266b, 366a, 366b) éloignés s'étendant de manière distale à partir de la partie proximale, le composant de mâchoires comportant en outre des première et seconde mâchoires (268a, 268b, 368a, 368b) disposées à des extrémités libres des premier et second bras éloignés, respectivement ; et
un manchon d'entraînement interne (280, 480) disposé de manière coulissante autour de l'ensemble effecteur d'extrémité (260, 360) à l'intérieur de la gaine externe (240), le manchon d'entraînement interne étant disposé de manière coulissante autour des premier et second bras (266a, 266b, 366a, 366b) éloignés et étant mobile d'une position proximale à une position distale pour amener les premier et second bras éloignés l'un vers l'autre, déplaçant ainsi les première et seconde mâchoires (268a, 268b, 368a, 368b) d'une position éloignée à une position rapprochée pour appliquer une agrafe chirurgicale autour du tissu disposé entre les première et seconde mâchoires,
**caractérisé en ce que** la gaine externe (240) comprend une paire de brides (248) s'étendant de manière distale à partir des côtés opposés du corps, les brides opposées définissant des butées de tissu (249) à des extrémités libres de celles-ci, les premier et second bras (266a, 266b, 366a, 366b) éloignés s'étendant de manière distale entre les brides opposées de la gaine externe.

2. Applicateur d'agrafes chirurgicales selon la revendication 1, le composant de mâchoires (262) étant formé de manière monolithique à partir d'une seule pièce de matériau.

3. Applicateur d'agrafes chirurgicales selon la revendication 1 ou 2, le composant de mâchoires (262) étant estampé.

4. Applicateur d'agrafes chirurgicales selon la revendication 2, la partie proximale (264) du composant de mâchoires étant épinglée à la gaine externe (240) par l'intermédiaire d'au moins une broche (250, 252).

5. Applicateur d'agrafes chirurgicales selon la revendication 4, l'au moins une broche (250, 252) s'étendant à travers des fentes opposées (286) définies à l'intérieur du manchon d'entraînement interne (280).

6. Applicateur d'agrafes chirurgicales selon l'une quelconque des revendications précédentes, les premier et second bras (266a, 266b) étant élastiquement flexibles d'une position relâchée à une position fléchie en réponse au mouvement du manchon d'entraînement interne (280) de la position proximale à la position distale pour déplacer ainsi les première et seconde mâchoires (268a, 268b) de la position éloignée à la position rapprochée.

7. Applicateur d'agrafes chirurgicales selon la revendication 1, le composant de mâchoires (362) comprenant des premier et second composants de mâchoire (362a, 362b) accouplés de manière pivotante l'un à l'autre et à la gaine externe (240) au niveau des parties proximales (364a, 364b) des premier et second composants de mâchoire, les premier et second composants de mâchoire comportant, respectivement, les premier et second bras (366a, 366b) s'étendant de manière distale à partir de leurs parties proximales respectives, et les première et seconde mâchoires (368a, 368b) étant disposées à des extrémités libres des premier et second bras, respectivement.

8. Applicateur d'agrafes chirurgicales selon la revendication 7, les premier et second composants de mâchoire (362a, 362b) étant identiques l'un à l'autre.

9. Applicateur d'agrafes chirurgicales selon la revendication 7 ou 8, les premier et second composants de mâchoire (362a, 362b) étant sensiblement rigides.

10. Applicateur d'agrafes chirurgicales selon la revendication 7, 8 ou 9, comprenant en outre un ressort à lames (374) disposé entre les premier et second bras (366a, 366b) conçu pour solliciter les première et seconde mâchoires (368a, 368b) vers la position éloignée.

11. Applicateur d'agrafes chirurgicales selon la revendication 10, le ressort à lames (374) comportant une charnière (378) et des première et seconde jambes (376a, 376b) s'étendant de manière distale à partir de la charnière, la première jambe étant en prise avec le premier bras (366a) et la seconde jambe étant en prise avec le second bras (366b).

12. Applicateur d'agrafes chirurgicales selon l'une quelconque des revendications 7 à 11, les parties proximales (364a, 364b) des premier et second composants de mâchoire (362a, 362b) étant accouplées de manière pivotante l'une à l'autre et à la gaine externe (240) par l'intermédiaire d'une broche (350), et la broche (350) s'étendant à travers des fentes opposées (286, 486) définies à l'intérieur du manchon d'entraînement interne (280, 480).

13. Applicateur d'agrafes chirurgicales selon l'une quelconque des revendications précédentes, le manchon d'entraînement interne (480) comportant une partie de corps distale (484) et une chape (490) s'étendant de manière distale à partir de la partie de corps distale, la chape comportant des première et seconde brides éloignées (492) et une broche (496) s'étendant transversalement entre les première et seconde brides, moyennant quoi, lors du retour du manchon d'entraînement interne (480) de la position distale à la position proximale, la broche (496) étant calée entre les premier et second bras (366a, 366b) pour pousser les première et seconde mâchoires (368a, 368b) de la position rapprochée à la position éloignée.

14. Applicateur d'agrafes chirurgicales selon la revendication 13, le manchon d'entraînement interne (480) définissant une configuration en coupe transversale rectangulaire ayant des côtés étroits opposés (485a) et des côtés larges opposés (485b).

15. Applicateur d'agrafes chirurgicales selon la revendication 14, les brides (492) de la chape (490) s'étendant à partir des côtés larges opposés (485b) du manchon d'entraînement interne (480).
